# EUROPÄISCHE PATENTSCHRIFT

(11) **EP 1 566 195 B1**
(45) Veröffentlichungstag und Bekanntmachung des Hinweises auf die Patenterteilung: **22.05.2013**
(21) Anmeldenummer: 05001544.5
(22) Anmeldetag: 26.01.2005
(51) Int. Cl.: A61M 5/00, A61M 5/50

(54) **Anordnung zum Lagern, Transportieren und Applizieren einer vorzugsweise medizinischen Flüssigkeit**
Device for storage, transport and administration of a preferably medical liquid
Dispositif pour le stockage, le transport et l'administration d'un liquide preferablement medical

(30) Priorität: 20.02.2004 DE 102004009918
(43) Veröffentlichungstag der Anmeldung: 24.08.2005
(73) Patentinhaber: Schott AG, 55122 Mainz (DE)
(72) Erfinder: Wolbring, Peter, Dr., 66606 St. Wendel (DE); Mosimann, Ralph, 9514 Wuppenau (CH)
(74) Vertreter: Duhme, Torsten

(56) Entgegenhaltungen:
- EP-A- 0 645 152
- US-A- 3 811 441
- US-A- 5 817 064
- US-A- 5 876 379
- US-A1- 2003 220 615

## Beschreibung

Die vorliegende Erfindung betrifft eine Anordnung zum Lagern, Transportieren und Applizieren einer vorzugsweise medizinischen Flüssigkeit. Ein besonders bevorzugter Anwendungsfall für eine derartige Anordnung sind Spritzen, die von einem pharmazeutischen Hersteller mit einem Medikament vorgefüllt werden, so dass die Applikation des Medikaments praktisch direkt aus der "Transportverpackung" erfolgen kann.

Die Erfindung betrifft ferner einen Originalitätsverschluss für eine vorzugsweise medizinische Spritze, der es ermöglicht, die Spritze nicht nur für die Applikation der Flüssigkeit, sondern auch als Anordnung der zuvor genannten Art, d.h. also für die Lagerung und den Transport, zu verwenden.

Die Bereitstellung und Verwendung von herstellerseitig vorgefüllten Spritzen ist im Stand der Technik bereits seit längerem bekannt. Es ist ferner bekannt, Spritzen mit einem Originalitätsverschluss zu versehen. So zeigt EP 0 389 938 A2 beispielsweise eine Spritze mit einem Spritzenzylinder, an dessen distalem Ende eine Nadelschutzkappe sitzt. Auf die Nadelschutzkappe ist eine Überwurfhülse aufgeschoben, deren proximales Ende mit dem Spritzenzylinder verklebt oder verschweißt ist. Nach dem Abdrehen der Überwurfhülse hält diese von sich aus nicht mehr auf der Nadelschutzkappe. Das Fehlen der Überwurfhülse zeigt damit an, dass der Originalitätsverschluss geöffnet wurde.

Des Weiteren ist es aus dieser Druckschrift bekannt, eine vergleichbare Überwurfhülse auch am proximalen Ende des Spritzenzylinders anzuordnen. Damit wird die eingeschobene Kolbenstange im Originalzustand gesichert. In diesem Fall kann die Spritze allerdings nicht vorgefüllt sein. Darüber hinaus ist diese Art des Originalitätsverschlusses sowohl bei der Herstellung als auch beim Öffnen relativ aufwendig und umständlich. Außerdem ist dieser Originalitätsverschluss relativ leicht zu manipulieren, indem die Überwurfhülse mit einem "versteckten" Klebstoffpunkt wieder in ihrer Originalposition gesichert wird.

Aus AT 394 950 B ist es bekannt, die Nadelschutzkappe einer Spritze mit einem Basisteil und einem Kappenteil auszubilden, wobei das Basisteil fest mit dem Spritzenzylinder verbunden wird und wobei zwischen dem Basisteil und dem Kappenteil eine Sollbruchstelle vorgesehen ist. Das Kappenteil besitzt ferner flügelartige Vorsprünge, die es ermöglichen, das Kappenteil vom Basisteil abzudrehen. Diese Art des Originalitätsverschlusses erfordert eine spezielle Ausbildung der Nadelschutzkappe, d.h. es ist hier nicht möglich, standardisierte Nadelschutzkappen zu verwenden. Außerdem schützt dieser Originalitätsverschluss nicht gegen Manipulationen am proximalseitigen Ende der Spritze.

Aus DE 43 27 171 C1 ist es bekannt, eine "konventionelle" Nadelschutzkappe mit einem Siegel zu versehen, das von außen auf die angrenzenden Bereiche der Nadelschutzkappe und des Spritzenzylinders in fließfähiger Form aufgebracht wird und anschließend aushärtet. Insbesondere wird die Verwendung eines Schmelzklebers als Siegel vorgeschlagen. Hierbei handelt es sich um eine einfache Art eines Originalitätsverschlusses, die jedoch nicht vollkommen manipulationssicher ist, da ein vergleichbarer Schmelzkleber auch nach dem erstmaligen Öffnen des Originalitätsverschlusses leicht wieder aufgebracht werden kann.

Weitere Originalitätsverschlüsse, mit denen das distale Ende einer Spritze gesichert werden kann, sind beispielsweise aus DE 197 51 219 A1, DE 39 16 101 A1, DE 199 55 652 A1 oder EP 0 440 047 A2 bekannt. In all diesen Fällen bezieht sich der Originalitätsverschluss allerdings nur auf das distale Ende der Spritze, d.h. das proximalseitige Spritzenende bleibt ungesichert.

Die Sicherung des proximalseitigen Endes einer Spritze durch einen Originalitätsverschluss ist beispielsweise aus DE 199 25 621 A1 bekannt. Hiernach wird auf der proximalseitigen Öffnung des Spritzenzylinders nach dem Einbringen des Kolbenstopfens eine Abreißfolie angebracht. In alternativen Ausführungsbeispielen wird vorgeschlagen, eine entsprechende Folie zwischen dem Spritzenzylinder und dem Kolbenstopfen bzw. zwischen dem Spritzenzylinder und der Kolbenstange anzuordnen. Auch für die Sicherung am distalen Ende der Spritze wird eine Abreißfolie vorgeschlagen, die zwischen einer distalen Abschlusskappe und dem Spritzenzylinder verläuft. Grundsätzlich kann mit derartigen Abreißfolien ein zuverlässiger Originalitätsverschluss erreicht werden. Das Aufbringen der Folien erfordert jedoch geeignete Verpackungsmaschinen, die nicht immer zur Verfügung stehen. Darüber hinaus kann das Entfernen der Abreißfolien im Einzelfall umständlich sein.

Aus DE 100 36 829 A1 ist es bekannt, am proximalen Ende eines Spritzenzylinders einen Rückanschlag auszubilden, der ein unbeabsichtigtes Herausziehen des Kolbenstopfens aus dem Spritzenzylinder verhindert. Konkret wird vorgeschlagen, das proximale Ende des Spritzenzylinders nach dem Einsetzen des Kolbenstopfens zu verengen, indem der Spritzenzylinder beispielsweise mit beheizten Zangen eingedrückt wird. In diesem Zusammenhang ist darauf hingewiesen, dass diese Art des Rückanschlags gleichzeitig auch einen Originalitätsverschluss darstellt, der sich allerdings nur auf das proximale Ende des Spritzenzylinders bezieht. Darüber hinaus kann auch diese Art von Originalitätsverschluss im Einzelfall schwierig in den Produktionsablauf bei einem pharmazeutischen Hersteller zu integrieren sein.

Darüber hinaus sind für medizinische Spritzen eine Vielzahl von anderweitig ausgeführten Rückanschlägen bekannt, die dazu dienen, ein unbeabsichtigtes Herausziehen des Kolbenstopfens aus dem Spritzenzylinder zu verhindern. Beispielhaft sei hierzu auf EP 0 738 517 B1, EP 0 764 450 B1, DE 44 34 644 C2 oder WO 94/13339 verwiesen. Ein zuverlässiger Originalitätsverschluss ist mit diesen bekannten Rückanschlägen jedoch nicht verbunden.

Aus EP 0 980 276 B1 oder US 4,540,405 ist es schließlich bekannt, eine medizinische Spritze mit einem Spritzenzylinder aus Glas, in einen unzerbrechlichen Haltekörper einzusetzen. Hierdurch soll es möglich sein, stark viskose Flüssigkeiten aus der Spritze herauszudrücken, ohne dass die Gefahr besteht, dass der Glaszylinder zerstört wird. Ein Originalitätsverschluss ist mit diesen Anordnungen jedoch nicht verbunden.

US 5,817,064 offenbart eine Anordnung nach dem Oberbegriff von Anspruch 1. Die bekannte Anordnung beinhaltet eine Schutzhülle, in der ein vorgefüllter Spritzenzylinder mit einem Kolbenstopfen und einer Nadel enthalten ist. Am distalen Ende der Schutzhülle ist eine Nadelschutzkappe angeordnet, die über Sollbruchstellen mit der Schutzhülle verbunden ist. Das proximale Ende der Schutzhülle ist mit einem Deckel verschlossen, der über weitere Sollbruchstellen mit der Schutzhülle verbunden ist. Vor Verwendung der Spritze wird der Deckel entfernt, und es wird eine Kolbenstange in den Kolbenstopfen eingeschraubt. Nach der Applikation kann die Spritze in die Schutzhülle zurückgezogen werden, um Verletzungen an der Nadel zu verhindern. Ein Rückanschlag, der eine Entnahme des Kolbenstopfens bei oder nach Verwendung der Spritze verhindert, ist nicht vorgesehen.

EP 6 645 152 A2 offenbart eine ähnliche Anordnung mit einer Spritze, die in einer beidseitig verschlossenen Schutzhülle angeordnet ist. Auch in diesem Fall ist kein Rückanschlag vorgesehen, der eine Entnahme des Kolbenstopfens bei oder nach Verwendung der Spritze verhindert.

Vor diesem Hintergrund ist es eine Aufgabe der vorliegenden Erfindung, eine Anordnung der eingangs genannten Art anzugeben, die einerseits kostengünstig hergestellt werden kann und die andererseits eine sichere Lagerung und einen sicheren Transport sowie eine einfache Applikation der Flüssigkeit ermöglicht.

Die genannte Aufgabe wird nach einem ersten Aspekt der Erfindung durch eine Anordnung nach Anspruch 1 gelöst.

Die vorliegende Erfindung geht damit den Weg, einen Originalitätsverschluss für eine Spritze dadurch zu realisiedass zwei endseitige Verschlusselemente über ein Verbindungselement so verbunden werden, dass sie sich gegenseitig in ihrer jeweiligen Position sichern. Um auch nur eines der beiden Verschlusselemente vom Spritzenzylinder zu entfernen, ist eine irreversible Trennung der beiden Verschlusselemente erforderlich, was durch die vorgesehene Sollbruchstelle begünstigt wird. Die praktische Realisierung ist mit geringem Aufwand verbunden, da die beiden Verschlusselemente für den Spritzenzylinder in aller Regel schon aus anderen Gründen vorhanden sind. Während die beiden Verschlusselemente bei den bislang bekannten Anordnungen in mindestens zwei Arbeitsgängen separat gesichert wurden, wenn überhaupt, genügt nun die Anbringung eines gemeinsamen Verbindungselements. Daher ist die neue Anordnung sehr einfach und kostengünstig zu realisieren.

Darüber hinaus wird durch die erfindungsgemäße Lösung ein sehr sicherer Originalitätsverschluss realisiert, da sich eine Manipulation stets auf beide Verschlusselemente auswirkt. Im Gegensatz dazu ist es bei den bislang bekannten Ansätzen grundsätzlich möglich, den Verschluss an einem Ende des Spritzenzylinders zu manipulieren, während das andere Ende vollkommen unversehrt bleibt. Außerdem führt die hier vorgeschlagene Lösung zu einem Originalitätsverschluss, der sofort auffällt, da sich das Verbindungselement zwangsläufig über einen beträchtlichen Teil des Spritzenzylinders erstreckt und somit von außen gut sichtbar ist.

Des weiteren kann das Verbindungselement, wie nachfolgend an Hand bevorzugter Ausführungsbeispiele gezeigt ist, sehr leicht so realisiert werden, dass es auf standardisierte oder zumindest weit verbreitete Verschlusselemente und Spritzenzylinder passt. Dabei ist ein mechanisches Verschließen der Anordnung möglich, so dass sich die neue Lösung sehr einfach in verbreitete und bewährte Herstellungsprozesse integrieren lässt und in hohen Stückzahlen möglich ist.

Außerdem bietet das am Spritzenzylinder verlaufende Verbindungselement eine einfache Möglichkeit, den Spritzenzylinder selbst vor mechanischen oder anderweitigen Einflüssen zu schützen, wie nachfolgend aufgezeigt wird. Die neue Anordnung bietet damit über den Manipulationsschutz hinaus die Möglichkeit, den Spritzenzylinder auch vor unbeabsichtigten Beschädigungen zu schützen.

Des weiteren ist der Rückanschlag als separates Bauteil am proximalen Ende des Spritzenzylinders befestigt. Diese Ausgestaltung ermöglicht es, konventionelle und damit weit verbreitete Spritzenzylinder für die neue Anordnung zu verwenden, wodurch die Herstellungskosten weiter reduziert sind. Ein weiterer Vorteil ist die Möglichkeit, Spritzenzylinder und Rückanschlag für ein späteres Recycling voneinander zu trennen. Auch hierdurch können die Gesamtkosten weiter reduziert werden.

Schließlich ist das proximale Verschlusselement mit Hilfe von Bauteilen realisiert, die auch für die spätere Applikation der Flüssigkeit verwendet werden. Hierdurch wird die Teilevielfalt der neuen Anordnung bzw. des neuen Originalitätsverschlusses reduziert. Die Anordnung lässt sich damit noch einfacher und kostengünstiger realisieren.

Insgesamt ermöglicht die neue Anordnung damit auf kostengünstige Weise einen sicheren Transport bzw. die sichere Lagerung einer Flüssigkeit, was insbesondere im medizinischen Bereich erwünscht ist. Die genannte Aufgabe ist damit vollständig gelöst.

In einer Ausgestaltung sind das distale Verschlusselement und das Verbindungselement einstückig miteinander verbunden.

Typischerweise muss das distale Verschlusselement bei Spritzen zur Applikation des Spritzeninhalts abgenommen werden, sei es, dass es sich um eine Nadelschutzkappe handelt oder dass das distale Verschlusselement durch eine Injektionsnadel ersetzt wird. Indem das distale Verschlusselement und das Verbindungselement einstückig ausgeführt sind, wird die Anzahl der Handhabungsschritte, die zur Applikation des Spritzeninhalts erforderlich ist, reduziert. Die Applikation wird hierdurch vereinfacht. Darüber hinaus führt diese Ausgestaltung zu einer weiter reduzierten Teilezahl, was eine weitere Reduktion der Herstellungskosten ermöglicht.

In einer alternativen Ausgestaltung sind das distale Verschlusselement und das Verbindungselement zwei getrennte Bauwobei das Verbindungselement das distale Verschlusselement zumindest teilweise umgreift.

Im Vergleich zur vorhergehenden Alternative führt diese Ausgestaltung zu einer größeren Teilezahl. Sie besitzt andererseits den Vorteil, dass die neue Anordnung mit standardisierten Verschlusselementen am distalen Ende des Spritzenzylinders ausgeführt werden kann, was ebenfalls eine Reduktion der Herstellungskosten ermöglicht. Darüber hinaus können vorhandene Spritzen mit distalen Verschlusselementen sehr einfach mit dem neuen Originalitätsverschluss nachgerüstet werden. Daher kann diese Ausgestaltung besonders einfach in vorhandene Herstellungsabläufe integriert werden.

In einer weiteren Ausgestaltung umgibt das Verbindungselement den Spritzenzylinder hülsen- oder sogar topfartig.

In beiden Fällen wird der Spritzenzylinder durch das Verbindungselement vor äußeren Beschädigungen geschützt, wodurch sowohl der Transport als auch die Lagerung einfacher und sicherer werden. Dies gilt insbesondere, wenn die Spritzenzylinder aus Glas hergestellt sind, was Vorteile in Bezug auf die Sterilisierbarkeit bietet.

In einer weiteren Ausgestaltung weist das Verbindungselement im Bereich des Spritzenzylinders zumindest eine fensterartige Ausnehmung auf.

Eine solche Ausnehmung ermöglicht einen Blick auf den Spritzenzylinder und seinen Inhalt selbst dann, wenn der Spritzenzylinder in dem hülsen- oder topfartigen Verbindungselement steckt.

Damit ist eine optische Kontrolle des Spritzeninhalts auch ohne Aufbrechen des Originalitätsverschlusses möglich. Außerdem führt eine solche Ausnehmung zu einer Gewichtsreduktion und damit auch zu einer Reduzierung der Transport-, Lager- und Herstellungskosten.

In einer weiteren Ausgestaltung weist das Verbindungselement an seiner Außenseite flügelartige Vorsprünge auf.

Derartige Vorsprünge erleichtern das Abtrennen des Verbindungselements von einem oder beiden Verschlusselementen. Die Applikation des Spritzeninhalts wird hierdurch weiter vereinfacht und beschleunigt.

In einer weiteren Ausgestaltung sind das proximale Verschlusselement und das Verbindungselement zweistückig ausgebildet, jedoch irreversibel miteinander verbunden. Die Verbindung der beiden Bauteile kann beispielsweise durch Kleben, Schweißen, Schrumpfen, Pressen, Verrasten und anderes erfolgen.

Diese Ausgestaltung ermöglicht ein besonders einfaches Anbringen und Verschließen des Originalitätsverschlusses. Dies gilt insbesondere, wenn das proximale Verschlusselement einen am Spritzenzylinder angeordneten Rückanschlag beinhaltet, der nach dem Einsetzen des Kolbenstopfens am Spritzenzylinder befestigt wird. Mit dieser Ausgestaltung ist eine besonders rationelle und einfache Fertigung möglich.

In einer besonders bevorzugten Ausgestaltung sind das proximale Verschlusselement und das Verbindungselement formschlüssig miteinander verbunden. Dies beinhaltet insbesondere eine Press-, Schrumpf- und/oder Rastverbindung.

Diese Ausgestaltung besitzt den Vorteil, dass das Schließen des Originalitätsverschlusses mechanisch erfolgt, was sehr einfach in vorhandene Herstellungsabläufe integriert werden kann.

In einer weiteren Ausgestaltung weist das Verbindungselement einen Ringabschnitt auf, der den Spritzenzylinder umgibt und dabei irreversibel in einer am proximalen Verschlusselement ausgebildeten Nut eingreift.

Diese Ausgestaltung ist besonders bevorzugt in Kombination mit einem Rückanschlag als Bestandteil des proximalen Verschlusselements. Durch den Ringabschnitt kann der Rückanschlag nämlich dauerhaft am Spritzenzylinder fixiert werden, so dass selbst nach dem Aufbrechen des Originalitätsverschlusses ein unbeabsichtigtes Herausziehen des Kolbenstopfens aus dem Spritzenzylinder verhindert ist. Dies ist besonders vorteilhaft, wenn hochgradig toxische und/oder sehr teure Medikamente in dem Spritzenzylinder abgefüllt sind.

Ausführungsbeispiele der Erfindung sind in der Zeichnung dargestellt und werden in der nachfolgenden Beschreibung näher erläutert. Es zeigen:
- Fig. 1: ein Ausführungsbeispiel der neuen Anordnung in einer perspektivischen Darstellung,
- Fig. 2: das Ausführungsbeispiel aus Fig. 1, jedoch ohne das dort verwendete topf- oder hülsenartige Verbindungselement,
- Fig. 3: das Ausführungsbeispiel aus Fig. 1 in einer Querschnittsansicht, und
- Fig. 4: eine Spritze zum Applizieren einer Flüssigkeit nach Aufbrechen des Originalitätsverschlusses aus dem Ausführungsbeispiel gemäß Fig. 1.

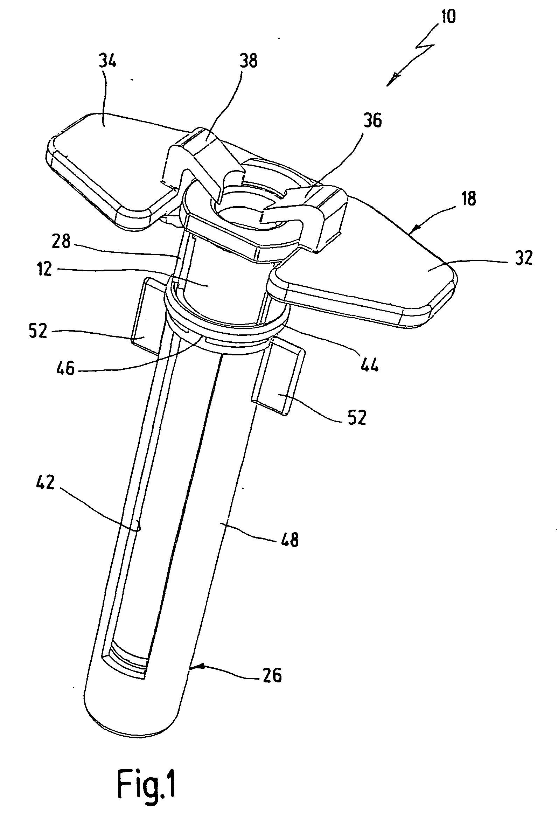

In den Fig. 1 bis 3 ist ein Ausführungsbeispiel einer erfindungsgemäßen Anordnung in seiner Gesamtheit mit der Bezugsziffer 10 bezeichnet.

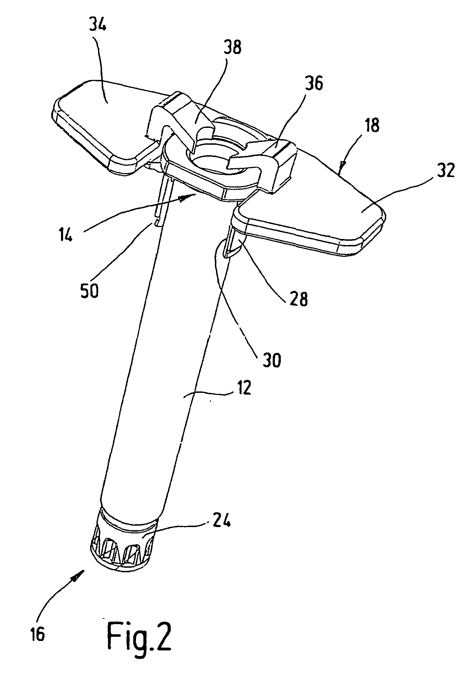

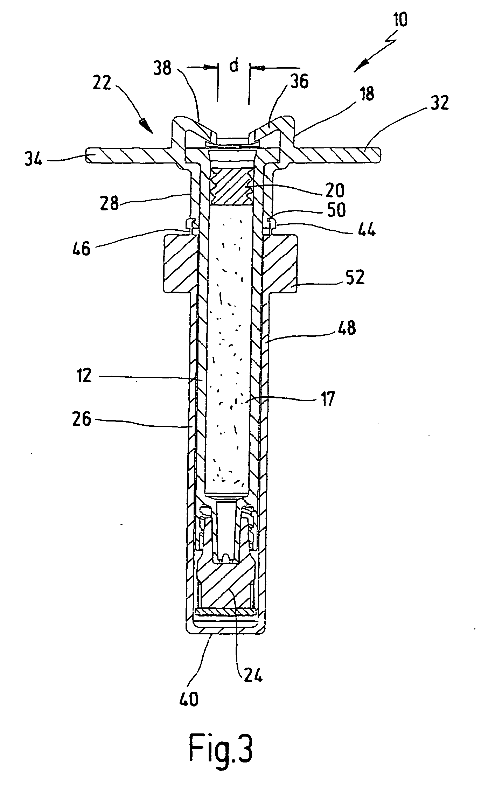

Die Anordnung 10 beinhaltet einen Spritzenzylinder 12 mit einem proximalen Ende 14 und einem distalen Ende 16 (siehe Fig. 2). Am proximalen Ende 14 ist ein Rückanschlag 18 für einen im Spritzenzylinder 12 angeordneten Kolbenstopfen 20 (Fig. 3) vorgesehen. Rückanschlag 18 und Kolbenstopfen 20 bilden in dem Ausführungsbeispiel gemeinsam ein proximales Verschlusselement, das in Fig. 3 insgesamt mit Bezugsziffer 22 bezeichnet ist.

Am distalen Ende 16 des Spritzenzylinders ist eine an sich bekannte Verschlusskappe 24 angeordnet. Statt dessen könnte hier jedoch beispielsweise auch ein Adapter für eine Nadel oder eine Nadel mit Nadelschutzkappe sitzen. Darüber hinaus kann die Verschlusskappe am distalen Ende 16 des Spritzenzylinders 12 auch eine andere Form aufweisen als hier gezeigt.

Mit der Bezugsziffer 26 ist ein Verbindungselement bezeichnet, das im vorliegenden Ausführungsbeispiel topfartig ausgebildet ist. Es umschließt den Spritzenzylinder 12 und die an seinem distalen Ende 16 angeordnete Verschlusskappe 24 und ist im Bereich des proximalen Endes 14 in nachfolgend näher beschriebener Weise mit dem Rückanschlag 18 verbunden. Zusammen bilden das proximale Verschlusselement 22 (hier mit Rückanschlag 18 und Kolbenstopfen 20), das distale Verschlusselement (hier die Verschlusskappe 24) und das Verbindungselement 26 einen Originalitätsverschluss für den Spritzenzylinder 12.

Der Rückanschlag 18 ist in diesem Ausführungsbeispiel ein separates Bauteil, das am proximalen Ende des Spritzenzylinders 12 befestigt ist. Hierzu besitzt der Rückanschlag 18 eine zylinderschalenförmige Wand 28, die den Spritzenzylinder 12 im Bereich seines proximalen Endes 14 fest umgreift. Ein seitlicher Öffnungsbereich 30 der Wand 28 ist dabei so dimensioniert, dass der Rückanschlag 18 nach Art einer Schnappverbindung seitlich auf das proximale Ende des Spritzenzylinders 12 aufgeschoben werden kann.

Am oberen Ende der Wand 28 besitzt der Rückanschlag 18 zwei seitlich abstehende Plattenbereiche 32, 34, die eine Fingerauflage für den Bediener der Spritze ausbilden. Der gezeigte Rückanschlag 18 ist damit vor allem für Spritzenzylinder vorgesehen, bei denen eine derartige Fingerauflage nicht am Spritzenzylinder ausgebildet ist. Grundsätzlich kann die vorliegende Erfindung jedoch auch bei solchen Spritzenzylindern angewendet werden, wobei der Rückanschlag 18 dann ohne oder mit entsprechend modifizierten Plattenbereichen 32, 34 ausgebildet ist.

Seitlich vom Spritzenzylinder 12 ist hier auf jedem der beiden Plattenbereiche 32, 34 ein krallenartiger Fanghaken 36, 38 angeordnet. Die Fanghaken 36, 38 weisen radial aufeinander zu. Ihre freien Enden sind zudem zum distalen Ende 16 des Spritzenzylinders 12 hingeneigt. Der lichte Abstand zwischen den beiden Fanghaken 36, 38 ist in Fig. 3 mit d angegeben. Er entspricht in etwa dem Außendurchmesser des Kolbenstopfens 20, so dass der Kolbenstopfen 20 beim Herausziehen aus dem Spritzenzylinder 12 in Anlage mit den Fanghaken 36, 38 kommt. Wird der Kolbenstopfen 20 dann weiter aus dem Spritzenzylinder 12 herausgezogen, verengt sich der lichte Abstand d zwischen den beiden Fanghaken 36, 38 auf Grund der eingestellter Neigung. Der Haltedruck am Kolbenstopfen 20 erhöht sich, so dass eine vollständige Entnahme des Kolbenstopfens 20 aus dem Spritzenzylinder 12 unterbunden ist.

In dem gezeigten und derzeit bevorzugten Ausführungsbeispiel ist der lichte Abstand d zwischen den beiden Fanghaken 36, 38 in etwa gleich dem lichten Innendurchmesser des Spritzenzylinders 12. Mit anderen Worten ragen die beiden Fanghaken 36, 38 nicht in eine projizierte Kreislinie hinein, die dem Innendurchmesser des Spritzenzylinders 12 entspricht. Da der Kolbenstopfen 20 in dem Spritzenzylinder jedoch radial zusammengepresst ist, um eine gute seitliche Abdichtung an der Zylinderinnenwand zu erreichen, können die beiden Fanghaken 36, 38 den Kolbenstopfen 20 trotzdem zuverlässig festhalten. Diese besondere Art der Ausbildung des Rückanschlags 18 ist Gegenstand einer weiteren Anmeldung der vorliegenden Anmelderin. Sie ist für die Realisierung der hiesigen Erfindung jedoch nicht zwingend erforderlich, d.h. die vorliegende Anordnung kann grundsätzlich auch mit anderen Arten von Rückanschlägen realisiert werden, wie sie beispielsweise aus dem eingangs genannten Stand der Technik bekannt sind.

Das Verbindungselement 26 ist in dem bevorzugten Ausführungsbeispiel topfartig ausgebildet, wobei der Spritzenzylinder 12 mit seiner distalen Verschlusskappe 24 passgenau in dem "Topf" steckt (Fig. 3). Abweichend hiervon kann das Verbindungselement 26 jedoch beispielsweise auch ohne Boden 40 ausgebildet sein, so dass es dann eher die Form einer Hülse besitzt. Wichtig ist in diesem Zusammenhang lediglich, dass das Verbindungselement 26 gewährleistet, dass die Verschlusskappe 24, allgemeiner ein am distalen Ende 16 angeordnetes Verschlusselement, nicht entfernt werden kann, solange das Verbindungselement 26 auf dem Spritzenzylinder 12 sitzt. Hierzu genügt beispielsweise ein umgebördelter Rand, eine Verdickung oder ein Steg, die am Verbindungselement 26 im Bereich der Verschlusskappe 24 ausgebildet sind.

Im bevorzugten Ausführungsbeispiel besitzt das Verbindungselement 26 zwei fensterartige Ausnehmungen 42, die parallel zueinander verlaufen, jedoch um 180° versetzt zueinander am Außenumfang des Verbindungselements 26 angeordnet sind. Dementsprechend ist die zweite Ausnehmung 42 in den Darstellungen in Fig. 1 und 3 nicht zu sehen.

An seinem oberen (proximalen) Ende besitzt das Verbindungselement 26 einen Ring 44, der über vier dünne Stege 46 mit dem hülsenartigen Körper 48 des Verbindungselements 26 verbunden ist. Die Stege 46 sind hier gleichmäßig am Außenumfang des Verbindungselements 26 verteilt. Sie bilden Sollbruchstellen, an denen das Verbindungselement 26 aufgebrochen und sodann der Körper vom Spritzenzylinder 12 abgenommen werden kann.

Der Ring 44 des Verbindungselements 26 sitzt im vorliegenden Ausführungsbeispiel passgenau in einer Nut 50, die am distalen Ende der Wand 28 ausgebildet ist. Ring 44 und Nut 50 bilden einen Rastverschluss, mit dem das Verbindungselement 26 und der Rückanschlag 18 zusammengefügt werden. Alternativ und/oder ergänzend könnte der Ring 44 mit dem Rückanschlag 18 auch verklebt, verschweißt oder in anderer Weise irreversibel verbunden sein.

Mit der Bezugsziffer 52 sind zwei flügelartige Vorsprünge bezeichnet, die knapp unterhalb des Rings 44 am Verbindungselement 26 ausgebildet sind. Die flügelartige Vorsprünge 52 ermöglichen ein einfaches Verdrehen des Verbindungselementes 26 relativ zum Rückanschlag 18, und zwar so, dass die Stege 46 aufbrechen. Dadurch, dass der Ring 44 in der Nut 50 am Rückanschlag 18 verbleibt, ist der Rückanschlag 18 weiterhin am proximalen Ende des Spritzenzylinders 12 gesichert, d.h. er kann ohne irreversible Zerstörung des Ringes 44 nicht entfernt werden.

Durch die bevorzugte Ausbildung des Verbindungselements 26 in Form einer Hülse oder eines Topfes wird der Spritzenzylinder 12 beim Transport und der Lagerung zusätzlich geschützt. Für die Realisierung des Originalitätsverschlusses ist diese Form des Verbindungselements 26 jedoch nicht unbedingt erforderlich. Insbesondere bei Spritzenzylindern 12, die aus Kunststoff hergestellt sind, kann das Verbindungselement 26 auch eine völlig andere Form besitzen, solange es die vorgesehene Funktion erfüllt, nämlich das proximale und distale Verschlusselement (hier also Rückanschlag 18 bzw. Verschlusskappe 24) in ihren jeweiligen Positionen zu sichern. Beispielsweise kann das Verbindungselement 26 ein einfacher Steg sein, der außerhalb des Spritzenzylinders 12 von der Verschlusskappe 24 bis zum Rückanschlag 18 verläuft. Vorzugsweise besteht das Verbindungselement aus einem steifen oder zumindest wenig dehnbaren Material, um eine Entnahme der Verschlusskappe und/oder des Rückanschlags 18 wirkungsvoll zu verhindern.

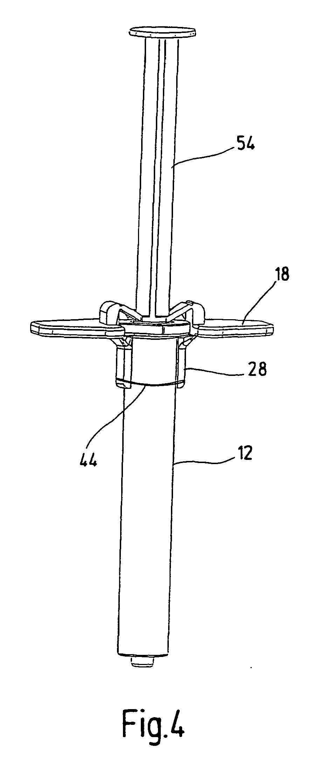

Fig. 4 zeigt den Spritzenzylinder 12 mit dem Rückanschlag 18 nach Entfernen des Verbindungselements 26 und der Verschlusskappe 24 und nach Einschrauben einer Kolbenstange 54 in den Kolbenstopfen 20. Die Kolbenstange 54 kann getrennt von der Anordnung 10 an den Endanwender mitgeliefert werden. Alternativ ist es auch möglich, die Kolbenstange 54 bereits beim Hersteller in den Kolbenstopfen 20 einzuschrauben oder anderweitig dort zu befestigen. Aus Gründen der Übersichtlichkeit ist die Kolbenstange 54 in den Fig. 1 bis 3 jedoch nicht dargestellt.

## Patentansprüche

1. Anordnung zum Lagern, Transportieren und Applizieren einer vorzugsweise medizinischen Flüssigkeit (17), mit
- einem Spritzenzylinder (12) zum Aufnehmen der Flüssigkeit (17), wobei der Spritzenzylinder (12) ein proximales (14) und ein distales (16) Ende aufweist,
- einem proximalen Verschlusselement (22) mit einem im Spritzenzylinder angeordneten Kolbenstopfen (20), der das proximale Ende (14) des Spritzenzylinders (12) verschließt,
- einem distalen Verschlusselement (24), das das distale Ende (16) des Spritzenzylinders (12) verschließt, und
- einem Verbindungselement (26), das das proximale und das distale Verschlusselement (22, 24) klammerartig miteinander verbindet,
- wobei zwischen den beiden Verschlusselementen (22, 24) und dem Verbindungselement (26) zumindest eine Sollbruchstelle (46) vorgesehen ist, die eine irreversible Trennung der beiden Verschlusselemente (22, 24) begünstigt,
**dadurch gekennzeichnet, dass** das proximale Verschlusselement (22) ferner einen Rückanschlag (18) beinhaltet, der als separates Bauteil an dem proximalen Ende (14) des Spritzenzylinders (12) befestigt ist und eine proximalseitige Entnahme des Kolbenstopfens (20) aus dem Spritzenzylinder (12) verhindert.

2. Anordnung nach Anspruch 1, **dadurch gekennzeichnet, dass** das distale Verschlusselement (24) und das Verbindungselement (26) einstückig miteinander verbunden sind.

3. Anordnung nach einem der Ansprüche 1 oder 2, **dadurch gekennzeichnet, dass** das distale Verschlusselement (24) und das Verbindungselement (26) zwei getrennte Bauteile sind, wobei das Verbindungselement (26) das distale Verschlusselement (24) zumindest teilweise umgreift.

4. Anordnung nach Anspruch 3, **dadurch gekennzeichnet, dass** das Verbindungselement (26) den Spritzenzylinder (12) hülsenartig umgibt.

5. Anordnung nach Anspruch 4, **dadurch gekennzeichnet, dass** das Verbindungselement (26) im Bereich des Spritzenzylinders (12) zumindest eine fensterartige Ausnehmung (42) aufweist.

6. Anordnung nach einem der Ansprüche 1 bis 5, **dadurch gekennzeichnet, dass** das Verbindungselement (26) an seiner Außenseite flügelartige Vorsprünge (52) aufweist.

7. Anordnung nach einem der Ansprüche 1 bis 6, **dadurch gekennzeichnet, dass** das proximale Verschlusselement (22) und das Verbindungselement (26) zweistückig ausgebildet, jedoch irreversibel miteinander verbunden sind.

8. Anordnung nach Anspruch 7, **dadurch gekennzeichnet, dass** das proximale Verschlusselement (22) und das Verbindungselement (26) formschlüssig miteinander verbunden sind.

9. Anordnung nach Anspruch 7 oder 8, **dadurch gekennzeichnet, dass** das Verbindungselement (26) einen Ringabschnitt (44) aufweist, der den Spritzenzylinder (12) umgibt und dabei irreversibel in eine am proximalen Verschlusselement (22) ausgebildete Nut (50) eingreift.

## Claims

1. An arrangement for storing, transporting and administering a preferably medical liquid (17), comprising
- a syringe barrel (12) for receiving the liquid (17), said syringe barrel (12) having a proximal end (14) and a distal end (16),
- a proximal closure element (22) comprising a plunger stopper (20) arranged in the syringe barrel for closing the proximal end (14) of the syringe barrel (12),
- a distal closure element (24) for closing the distal end (16) of the syringe barrel (12), and
- a connection element (26) which connects the proximal and distal closure elements (22, 24) to one another in a clip-like fashion,
- wherein at least one predetermined breaking point (46) is provided between the two closure elements (22, 24) and the connection element (26), and said predetermined breaking point favours an irreversible separation of the two closure elements (22, 24),
**characterized in that** the proximal closure element (22) further comprises a backstop (18) which is secured as a separate structural part on the proximal end (14) of the syringe barrel (12) preventing proximal removal of the plunger stopper (20) from the syringe barrel (12).

2. The arrangement according to Claim 1, **characterized in that** the distal closure element (24) and the connection element (26) are connected integrally to one another.

3. The arrangement according to one of Claims 1 or 2, **characterized in that** the distal closure element (24) and the connection element (26) are two separate structural parts, the connection element (26) engaging at least partially around the distal closure element (24).

4. The arrangement according to Claim 3, **characterized in that** the connection element (26) surrounds the syringe barrel (12) in a sleeve-like fashion.

5. The arrangement according to Claim 4, **characterized in that** the connection element (26) has at least one window-like recess (42) in the area of the syringe barrel (12).

6. The arrangement according to one of Claims 1 to 5, **characterized in that** the connection element (26) has wing-like projections (52) on its outside.

7. The arrangement according to one of Claims 1 to 6, **characterized in that** the proximal closure element (22) and the connection element (26) are designed as two pieces but are connected to one another in an irreversible manner.

8. The arrangement according to Claim 7, **characterized in that** the proximal closure element (22) and the connection element (26) are connected to one another with a form fit.

9. The arrangement according to Claim 7 or 8, **characterized in that** the connection element (26) has a ring portion (44) which surrounds the syringe barrel (12) and engages irreversibly in a groove (50) formed on the proximal closure element (22).

## Revendications

1. Dispositif pour le stockage, le transport et l'administration d'un liquide de préférence médical (17), avec
- un cylindre de seringue (12) destiné à contenir le liquide (17), dans lequel le cylindre de seringue (12) présente une extrémité proximale (14) et une extrémité distale (16),
- un élément de fermeture proximal (22) avec un bouchon formant piston (20) disposé dans le cylindre de seringue, qui ferme l'extrémité proximale (14) du cylindre de seringue (12),
- un élément de fermeture distal (24), qui ferme l'extrémité distale (16) du cylindre de seringue (12),
et
- un élément de liaison (26), qui relie l'un à l'autre à la manière d'une attache les éléments de fermeture proximal et distal (22, 24),
- dans lequel il est prévu entre les deux éléments de fermeture (22, 24) et l'élément de liaison (26) au moins un point de rupture (46), qui favorise une séparation irréversible des deux éléments de fermeture (22, 24),
**caractérisé en ce que** l'élément de fermeture proximal (22) comporte en outre une butée arrière (18), qui est fixée comme composant séparé à l'extrémité proximale (14) du cylindre de seringue (12) et qui empêche un enlèvement côté proximal du bouchon formant piston (20) hors du cylindre de seringue (12).

2. Dispositif selon la revendication 1, **caractérisé en ce que** l'élément de fermeture distal (24) et l'élément de liaison (26) sont assemblés l'un à l'autre d'un seul tenant.

3. Dispositif selon l'une des revendications 1 ou 2, **caractérisé en ce que** l'élément de fermeture distal (24) et l'élément de liaison (26) sont deux composants séparés, dans lequel l'élément de liaison (26) entoure au moins en partie l'élément de fermeture distal (24).

4. Dispositif selon la revendication 3, **caractérisé en ce que** élément de liaison (26) entoure le cylindre de seringue (12) à la manière d'une douille.

5. Dispositif selon la revendication 4, **caractérisé en ce que** l'élément de liaison (26) présente au moins un évidement de type fenêtre (42) dans la région du cylindre de seringue (12).

6. Dispositif selon l'une quelconque des revendications 1 à 5, **caractérisé en ce que** l'élément de liaison (26) présente des saillies en forme d'ailettes (52) sur sa face extérieure.

7. Dispositif selon l'une quelconque des revendications 1 à 6, **caractérisé en ce que** l'élément de fermeture proximal (22) et l'élément de liaison (26) sont réalisés en deux parties, mais sont assemblés l'un à l'autre de façon irréversible.

8. Dispositif selon la revendication 7, **caractérisé en ce que** l'élément de fermeture proximal (22) et l'élément de liaison (26) sont assemblés l'un à l'autre par emboîtement.

9. Dispositif selon la revendication 7 ou 8, **caractérisé en ce que** l'élément de liaison (26) présente une partie annulaire (44), qui entoure le cylindre de seringue (12) et qui s'engage en l'occurrence de façon irréversible dans une rainure (50) formée sur l'élément de fermeture proximal (22).
